# EUROPEAN PATENT SPECIFICATION

(11) **EP 1 857 083 B1**
(45) Date of publication and mention of the grant of the patent: **29.10.2008**
(21) Application number: 07108294.5
(22) Date of filing: 15.05.2007
(51) Int. Cl.: A61F 9/007

(54) **Surgical system having integral pneumatic manifolds**
Chirurgisches System mit integrierten pneumatischen Übergangsstücken
Système chirurgical ayant des collecteurs pneumatiques intégrés

(30) Priority: 19.05.2006 US 437301
(43) Date of publication of application: 21.11.2007
(73) Proprietor: Alcon, Inc., 6331 Hünenberg (CH)
(72) Inventor: King, Nicolei R., Aliso Viejo, CA California 92656 (US); Williams, David L., Newport Beach, CA California 92663 (US)
(74) Representative: Hanna, Peter William Derek

(56) References cited:
- EP-A- 1 568 339
- US-A- 5 107 899
- US-A- 5 499 969
- US-A- 5 549 139

## Description

### Background of the Invention

The present invention relates generally to surgical systems and more specifically to surgical systems that control pneumatic devices.

Many microsurgical procedures require precision cutting and/or removal of various body tissues. For example, certain ophthalmic surgical procedures require the cutting and/or removal of the vitreous humor, a transparent jelly-like material that fills the posterior segment of the eye. The vitreous humor, or vitreous, is composed of numerous microscopic fibers that are often attached to the retina. Therefore, cutting and removal of the vitreous must be done with great care to avoid traction on the retina, the separation of the retina from the choroid, a retinal tear, or, in the worst case, cutting and removal of the retina itself.

Conventional vitrectomy probes typically include a hollow outer cutting member, a hollow inner cutting member arranged coaxially with and movably disposed within the hollow outer cutting member, and a port extending radially through the outer cutting member near the distal end thereof. Vitreous humor is aspirated into the open port, and the inner member is actuated, closing the port. Upon the closing of the port, cutting surfaces on both the inner and outer cutting members cooperate to cut the vitreous, and the cut vitreous is then aspirated away through the inner cutting member. This cutting action may be made using an electric cutter, but pneumatically driven probes operating at a relatively high pressure are more common.

Additionally, during typical ophthalmic procedures, air may be introduced into the posterior chamber. This air must be of relatively low pressure.

Conventional ophthalmic surgical instrument systems use vacuum to aspirate the surgical site and positive pressure to irrigate the site. Typically, a cassette is serially connected between the means used to generate pressure and the surgical instrument, for example, as described in US-A-5,499,969 (Nestlé, S.A.). The use of cassettes with surgical instruments to help manage irrigation and aspiration flows at a surgical site is well known. Aspiration fluid flow rate, vacuum level, irrigation fluid pressure, and irrigation fluid flow rate are some of the parameters that require precise control during ophthalmic surgery. For aspiration instruments, the air pressure is below atmospheric pressure, and fluid is removed from the surgical site. For irrigation instruments, the air pressure is higher than atmospheric pressure, and the fluid will be transported from the irrigation fluid reservoir to the surgical site.

Prior art surgical systems have controlled the various air pressures needed during surgery by using individual manifolds for each sub-system. For example, the aspiration subsystem typically included a manifold having the various required air passages needed to route the vacuum where needed, but the aspiration pump, such as a venturi pump, is mounted remote from the aspiration manifold. This type of construction, typified by US-A-5,549,139 (Storz Instrument Company), required that the various subsystems be connected via pneumatic tubing, with the tubing being interrupted at the required valves and "T's". This sort of construction increases assembly costs, the overall size of the system and can affect reliability.

Accordingly, a need continues to exist for a surgical system having a simplified construction.

### Brief Summary of the Invention

The present invention improves upon prior art by providing a surgical system having all of the various pneumatic control sub-systems integrally mounted on a common manifold, in accordance with claims which follow. The various required control mechanisms such as valves are likewise integrally mounted to the common manifold.

One objective of the present invention is to provide a surgical system having integrated pneumatic sub-systems.
Another objective of the present invention is to provide a surgical system having pneumatic sub-systems mounted on a common manifold.

Yet another objective of the present invention is to provide a surgical system for controlling pneumatic surgical devices.

These and other advantages and objectives of the present invention will become apparent from the detailed description, drawings and claims that follow.

### Brief Description of the Drawings

FIG. 1 is a front perspective view of a surgical console that may use the integral pneumatic manifold of the present invention.
FIG. 2 is a front perspective view of a cassette that may be used with the present invention.
FIG. 3 is a rear perspective view of a cassette that may be used with the present invention.
FIG. 4 is an exploded perspective view of the integral pneumatics manifold of the present invention.
FIG. 5 is an enlarged perspective view of the valve or pincher manifold of the present invention and illustrating several active mechanical elements mounted on the manifold.

### Detailed Description of the Preferred Embodiments

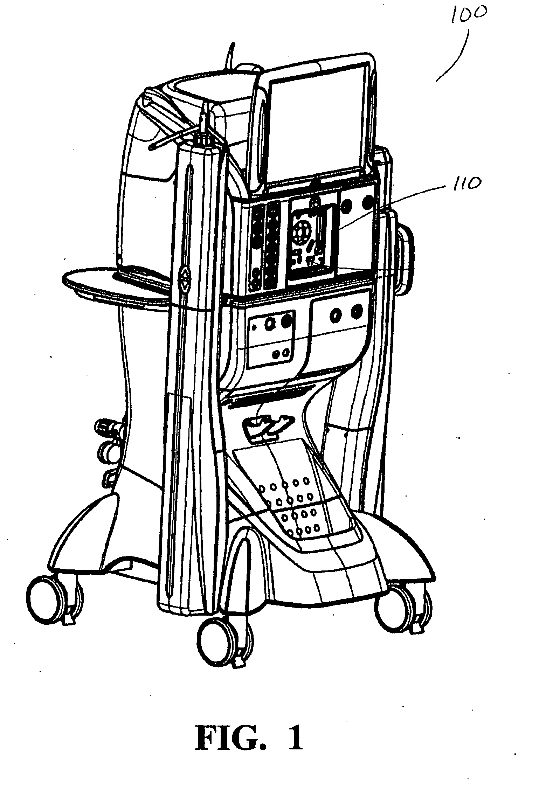

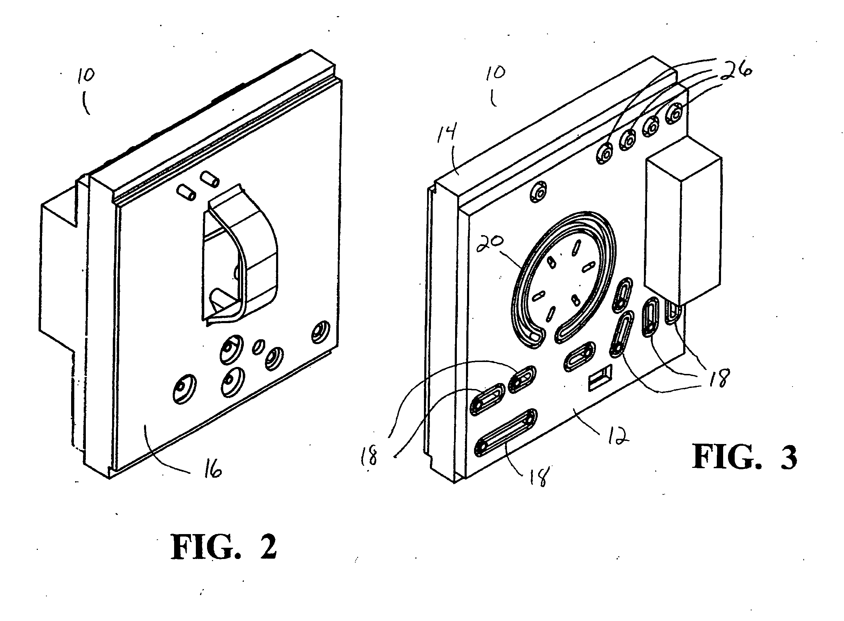

As best seen in FIGS. 1, 2 and 3, cassette 10 that may be used with the present invention generally included valve plate 12, body 14 and cover 16. Valve plate 12, body 14 and cover 16 may all be formed of a suitable, relatively rigid, and thermoplastic material. Valve plate 12 contains a plurality of openings 18 and pumping channel 20 that are sealed fluid tight by elastomers, forming a plurality of fluid paths. Ports 26 provide connectors between cassette 10 and surgical console 100 for the various irrigation and aspiration (pneumatic) functions of cassette 10 when cassette 10 is installed in cassette receiving portion 110 of console 100.

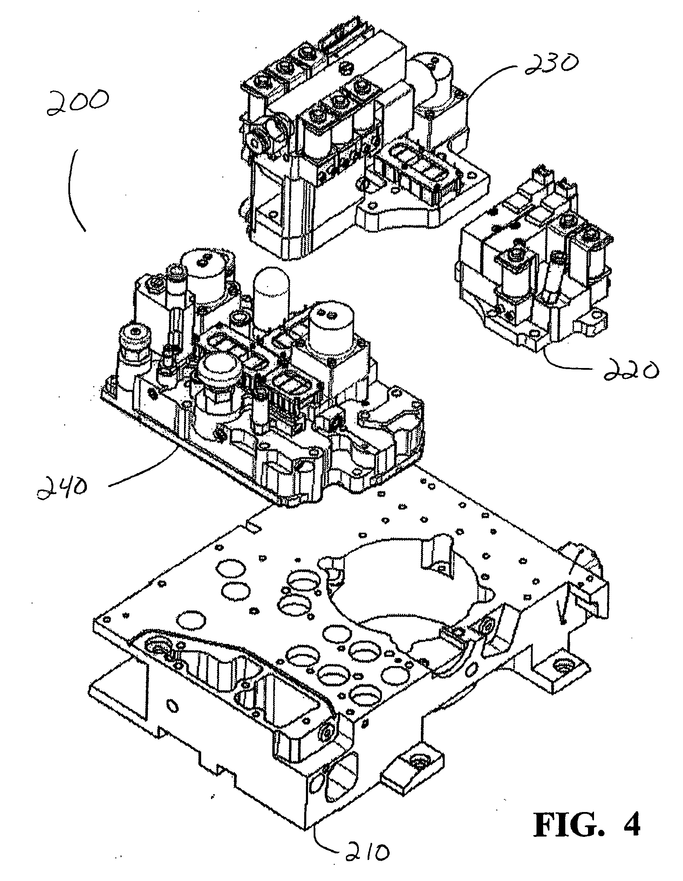

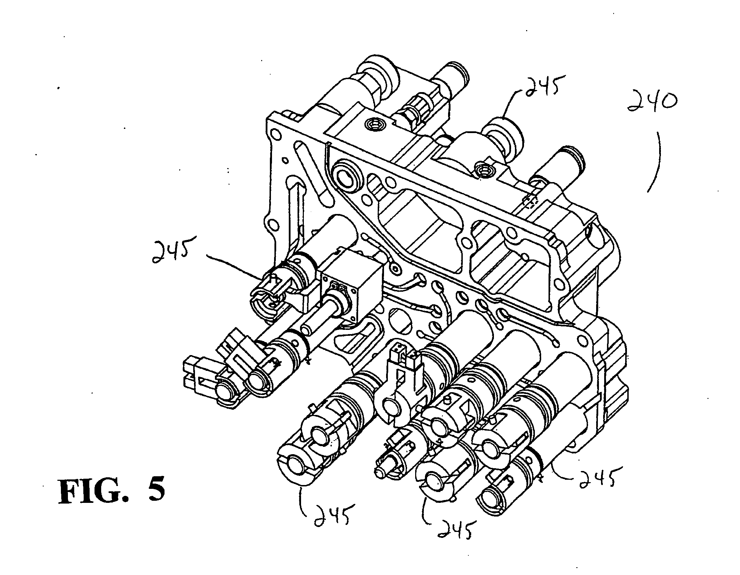

As best seen in FIG. 4, fluidics manifold 200 contains a plurality of sub-assemblies or manifolds mounted to common primary manifold 210. For example, fluidics manifold 200 may additionally contain aspiration manifold 220, and/or infusion/irrigation manifold 230 and/or valve or pincher manifold 240. As seen in FIG. 5, for example, each of manifolds 210, 220,230 and 240 (manifold 240 used as an illustrative example) are self-contained, and may contain necessary the valves, regulators, sensors or other active embedded mechanical, electrical or electromechanical devices required to perform each manifold's primary function, such as air cylinders 245, by way of example. Manifolds 220 and 230 pneumatically and fluidly communicate with cassette 10 through primary manifold 210, such communication being controlled by pincher manifold 240. Primary manifold 210 may be mounted in cassette receiving portion 110 of console 100 so that cassette 10 may be fluidly coupled to primary manifold 210. Primary manifold 210 may additionally contain pumps and fluid level and/or fluid flow sensors (all not shown).
Such a construction allows for the separation of the primary functionalities of each sub-assembly onto specific manifolds, thereby providing convenient and fast assembly, troubleshooting and repair. In addition, such a construction eliminates the various tubings and tubing connectors used in the prior art to connect the various components in each subassembly and reduces the overall size of the completed assembly.

This description is given for purposes of illustration and explanation. It will be apparent to those skilled in the relevant art that modifications may be made to the invention as herein described without departing from its scope.

## Claims

1. An ophthalmic surgical system (200), comprising a surgical console (100) having a cassette-receiving portion (110) adapted to receive a cassette (10) for managing irrigation and aspiration fluid flows by means of pneumatic sub-systems, comprising
a) a common primary fluidics and pneumatics manifold (210)
b) an infusion manifold (230)
c) an aspiration manifold (220)
**characterized in that**
a) the common primary fluidics and pneumatics manifold (210) is adapted to be mounted in the cassette-receiving portion (110) of the surgical console (100) so that the cassette may be fluidly coupled to the console;
b) the infusion manifold (230) is mounted on the primary manifold;
c) the aspiration manifold (220) is mounted on the primary manifold, wherein the aspiration manifold (220) and the infusion manifold (230) are adapted to pneumatically communicate with each other and with the cassette (10) through the primary manifold.

2. The surgical system of claim 1, further comprising a pincher manifold (240) mounted to the primary manifold (210), wherein the aspiration manifold (220) and the infusion manifold (230) are adapted to pneumatically communicate with each other through the pincher manifold.

3. The surgical system of claim 1, wherein the aspiration manifold (220) and the infusion manifold (230) are adapted to fluidly communicate with each other through the primary manifold (210).

4. The surgical system of claim 2, wherein the aspiration manifold (220), the infusion manifold (230) and the pincher manifold (240) are adapted to fluidly communicate with each other through the primary manifold (210).

5. The surgical system of claim 2, wherein the aspiration manifold (220) and the infusion manifold (230) are adapted to fluidly communicate with each other in use through a cassette (10), when a cassette is installed in the cassette-receiving portion (110) of the surgical console (100).

6. The surgical system of claim 1, further comprising at least one active embedded device (245) mounted on the aspiration manifold (220) and/or the infusion manifold (230).

7. The surgical system of claim 2, further comprising at least one active embedded device (245) mounted on the aspiration manifold (220) and/or the infusion manifold (230) and/or the pincher manifold (240) and/or the primary manifold (210).

8. The surgical system of any one of claims 6 to 7, wherein the active embedded device (245) comprises any mechanical, electrical, or electromechanical device adapted to perform the respective manifold's primary function, including a valve, regulator, sensor and air cylinder.

## Patentansprüche

1. Augenoperationssystem (200), welches eine Operationskonsole (100) enthält, welche einen Kassettenaufnahmeabschnitt (110) hat, welcher dazu ausgelegt ist, um eine Kassette (10) zum Verwalten von Spül- und Absaug-Fluidflüssen mittels eines pneumatischen Teilsystems aufzunehmen, welches enthält:
a) einen allgemeinen grundlegenden Fluid- und Pneumatik-Verteiler (210),
b) einen Infusions-Verteiler (230),
c) einen Absaug-Verteiler (220),
**dadurch gekennzeichnet, dass**
a) der allgemeine grundlegende Fluid- und Pneumatik-Verteiler (210) dazu ausgelegt ist, um in dem Kassettenaufnahmeabschnitt (110) von der Operationskonsole (100) befestigt zu werden, so dass die Kassette in Fluidverbindung zu der Konsole steht;
b) der Infusions-Verteiler (230) an dem grundlegenden Verteiler befestigt ist;
c) der Absaug-Verteiler (220) am grundlegenden Verteiler befestigt ist, wobei der Absaug-Verteiler (220) und der Infusions-Verteiler (230) dazu ausgelegt sind, über den grundlegenden Verteiler pneumatisch untereinander und mit der Kassette (10) in Verbindung zu stehen.

2. Operationssystem nach Anspruch 1, welches ferner einen Pincher-Verteiler (240) enthält, welcher an dem grundlegenden Verteiler (210) befestigt ist, wobei der Absaug-Verteiler (220) und der Infusions-Verteiler (230) dazu ausgelegt sind, über den Pincher-Verteiler pneumatisch untereinander in Verbindung zu stehen.

3. Operationssystem nach Anspruch 1, bei welchem der Absaug-Verteiler (220) und der Infusions-Verteiler (230) dazu ausgelegt sind, über den grundlegenden Verteiler (210) untereinander in Fluidverbindung zu stehen.

4. Operationssystem nach Anspruch 2, bei welchem der Absaug-Verteiler (220), der Infusions-Verteiler (230) und der Pincher-Verteiler (240) dazu ausgelegt sind, über den grundlegenden Verteiler (210) untereinander in Fluidverbindung zu stehen.

5. Operationssystem nach Anspruch 2, bei welchem der Absaug-Verteiler (220) und der Infusions-Verteiler (230) dazu ausgelegt sind, in der Verwendung über eine Kassette (10) untereinander in Fluidverbindung zu stehen, wenn eine Kassette in dem Kassettenaufnahmeabschnitt (110) von der Operationskonsole (100) eingesetzt ist.

6. Operationssystem nach Anspruch 1, welches ferner zumindest eine aktive eingebettete Vorrichtung (245) enthält, welche an dem Absaug-Verteiler (220) und/oder dem Infusions-Verteiler (230) befestigt ist.

7. Operationssystem nach Anspruch 2, welches ferner zumindest eine aktive eingebettete Vorrichtung (245) enthält, welche an dem Absaug-Verteiler (220) und/oder dem Infusions-Verteiler (230) und/oder dem Pincher-Verteiler (240) und/oder dem grundlegenden Verteiler (210) befestigt ist.

8. Operationssystem nach Anspruch 6 oder 7, bei welchem die aktive eingebettete Vorrichtung (245) eine jegliche mechanische, elektrische oder elektromechanische Vorrichtung enthält, welche dazu ausgelegt ist, um eine jeweilige Verteiler-Hauptfunktion durchzuführen, welche ein Ventil, einen Regler, einen Sensor und einen Luftzylinder enthält.

## Revendications

1. Système chirurgical ophtalmique (200), comprenant une console chirurgicale (100) ayant une partie de réception de cassette (110) adaptée pour recevoir une cassette (10) pour gérer des écoulements de fluide d'irrigation et d'aspiration par l'intermédiaire de sous-systèmes pneumatiques, comportant
a) un collecteur fluidique et pneumatique principal commun (210)
b) un collecteur de perfusion (230)
c) un collecteur d'aspiration (220)
**caractérisé en ce que**
a) le collecteur fluidique et pneumatique principal commun (210) est adapté pour être monté dans la partie de réception de cassette (110) de la console chirurgicale (100) de telle sorte que la cassette peut être couplée à la console de manière fluide,
b) le collecteur de perfusion (230) est monté sur le collecteur principal,
c) le collecteur d'aspiration (220) est monté sur le collecteur principal, le collecteur d'aspiration (220) et le collecteur de perfusion (230) étant adaptés pour communiquer de manière pneumatique l'un avec l'autre et avec la cassette (10) à travers le collecteur principal.

2. Système chirurgical selon la revendication 1, comportant en outre un collecteur à pinces (240) monté sur le collecteur principal (210), dans lequel le collecteur d'aspiration (220) et le collecteur de perfusion (230) sont adaptés pour communiquer de manière pneumatique l'un avec l'autre à travers le collecteur à pinces.

3. Système chirurgical selon la revendication 1, dans lequel le collecteur d'aspiration (220) et le collecteur de perfusion (230) sont adaptés pour communiquer de manière hydraulique l'un avec l'autre à travers le collecteur principal (210).

4. Système chirurgical selon la revendication 2, dans lequel le collecteur d'aspiration (220), le collecteur de perfusion (230) et le collecteur à pinces (240) sont adaptés pour communiquer de manière hydraulique les uns avec les autres à travers le collecteur principal (210).

5. Système chirurgical selon la revendication 2, dans lequel le collecteur d'aspiration (220) et le collecteur de perfusion (230) sont adaptés pour communiquer de manière hydraulique l'un avec l'autre en utilisation à travers une cassette (6), lorsqu'une cassette est installée dans la partie de réception de cassette (110) de la console chirurgicale (100).

6. Système chirurgical selon la revendication 1, comportant en outre au moins un dispositif intégré actif (245) monté sur le collecteur d'aspiration (220) et/ou le collecteur de perfusion (230).

7. Système chirurgical selon la revendication 2, comportant en outre au moins un dispositif intégré actif (245) monté sur le collecteur d'aspiration (220) et/ou le collecteur de perfusion (230) et/ou le collecteur à pinces (240) et/ou le collecteur principal (210).

8. Système chirurgical selon l'une quelconque des revendications 6 et 7, dans lequel le dispositif intégré actif (245) comprend un quelconque dispositif mécanique, électrique ou électromécanique, adapté pour remplir la fonction principale de collecteur respective, y compris une vanne, un régulateur, un capteur et un vérin pneumatique.
